# EUROPEAN PATENT SPECIFICATION

(11) **EP 0 896 524 B1**
(45) Date of publication and mention of the grant of the patent: **09.07.2003**
(21) Application number: 97921397.2
(22) Date of filing: 25.04.1997
(51) Int. Cl.: A61K 7/50, A61K 7/48

(54) **TOPICAL COMPOSITIONS COMPRISING DISPERSED SURFACTANT COMPLEX**
TOPISCHE ZUSAMMENSETZUNG MIT DISPERGIERTEM TENSIDKOMPLEX
COMPOSITIONS TOPIQUES COMPORTANT UN COMPLEXE DE TENSIO-ACTIFS DISPERSES

(30) Priority: 02.05.1996 US 641969
(43) Date of publication of application: 17.02.1999
(73) Proprietor: THE PROCTER & GAMBLE COMPANY, Cincinnati, Ohio 45202 (US)
(72) Inventor: ALBACARYS, Lourdes, Dessus, West Chester, OH 45069 (US); DECKNER, George, Endel, Cincinnati, OH 45249 (US); LISTRO, Joseph, Anthony, Mason, OH 45040 (US); MCATEE, David, Michael, Mason, OH 45040 (US)
(74) Representative: Clemo, Nicholas Graham
(86) International application number: US9707012
(87) International publication number: WO97040816

(56) References cited:
- EP-A- 0 015 032
- EP-A- 0 708 175
- WO-A-96/02225
- WO-A-97/03647
- DE-A- 19 544 156

## Description

### TECHNICAL FIELD

The present invention relates to topical compositions containing skin active agents in combination with a dispersed surfactant complex. This dispersed surfactant complex is formed prior to or during formulation of the composition, and comprises an anionic surfactant and a cationic surfactant. These topical compositions are useful for clarifying the skin and reducing pore size, while providing improved deposition of skin active agents, milder cleansing of the skin, and improved skin feel benefits.

### BACKGROUND OF THE INVENTION

Skin problems such as dry skin, psoriasis, ichtyosis, dandruff, callus, photodamaged skin, aged skin, and sunburn can be described as disorders of keratinization in which the shedding of stratum corenum cells at the skin surface is altered relative to normal, young, healthy skin. Such alteration results in shedding of large clusters of cells leading to visible scaling of the skin, a build-up of keratinaceous material on the surface or in follicles or ducts, and a rough texture to the skin surface. These conditions can be improved by desquamation, which is the removal of the outermost keratinaceous material.

There are many topical compositions that are directed to the treatment of individuals afflicted with various skin diseases such as those mentioned hereinbefore. These compositions are topically applied to the skin surface to provide delivery of the active agent to the affected areas, thereby promoting the desquamation process. Many such compositions such as those disclosed in EP 0 708 175 A1, however, are harsh and irritating to the skin, especially when combined with a surfactant in a cleansing composition. With respect to cleansing or rinse-off compositions containing skin active ingredients, efficient deposition of the skin active ingredient onto the skin during cleansing or rinse-off application has historically been difficult to achieve.

It has now been found that skin care compositions which comprise a combination of a skin active agent and a dispersed surfactant complex, wherein the dispersed complex is formed prior to or during formulation of the composition and comprises anionic and cationic surfactants, provides excellent topical delivery of the skin active agent. It has also been found that this dispersed surfactant complex, when incorporated into the topical composition defined herein, is especially gentle and nonirritating to the skin when compared to similar surfactant systems containing only free surfactant. The surfactant complex system described herein provides enhanced delivery or deposition of the skin active agents to the surface of the skin during cleansing or rinse-off application. The surfactant components of the dispersed complex remain effective for cleansing the skin and for promoting the desquamation process, but because the charges on some or all of individual surfactants are complexed, the surfactants are rendered less harsh and irritating to the skin versus similar systems comprising only the free surfactants.

In view of the foregoing, it is an object of the present invention to provide a topical composition for the enhanced delivery of skin active agents to the skin, and further to provide for topical delivery of skin clarifying agents to the skin, and further to provide for such topical delivery in a coacervate system that provides milder, less irritating cleansing of skin, and further to provide for such a topical delivery system that also renders the skin soft and smooth feeling.

### SUMMARY OF THE INVENTION

The present invention is directed to topical skin care compositions which comprise from about 0.1% to about 20% by weight of a cationic surfactant, from about 0.1% to about 20% by weight of an anionic surfactant, from about 0.001% to about 20% by weight of a skin active agent, from about 40% to about 99% by weight of water, and dispersed surfactant complex formed prior to or during formulation of the composition, which complex comprises from about 25% to about 100% of the cationic surfactant of the composition and from about 25% to about 100% of the anionic surfactant of the composition. These skin care compositions provide enhanced deposition of skin active agents onto the surface of skin, milder cleansing of skin, and improved skin feel benefits.

The present invention is also directed to methods of using the topical compositions of the present invention for treating skin with skin active agents, for cleansing skin, and for clarifying skin and reducing pore size.
The present invention is also directed to methods for preparing the compositions, which methods comprise the steps of (a) combining an aqueous solution of the cationic surfactant and an aqueous solution of the anionic surfactant to form an aqueous dispersion of a surfactant complex comprising cationic and anionic surfactant, and then (b) combining the aqueous dispersion with the skin active agent.

### DETAILED DESCRIPTION OF THE INVENTION

The topical compositions of the present invention are useful for delivering a wide variety of active ingredients to the surface of the skin. The topical compositions of the present invention are also useful for clarifying the skin and reducing pore size. These compositions are applied directly to the skin to provide the desired delivery of active ingredients, or they are used in a cleansing process to provide topical delivery of active ingredients during cleansing.

It is believed that the cationic surfactant component of the topical compositions complexes with the anionic surfactant component, forming a highly stable, dispersed surfactant complex relative to the individual surfactant components. The dispersed surfactant complex, which may or may not be in the form of a visible coacervate phase, is especially useful for aiding in the delivery and deposition of the skin active agent to the skin, thereby aiding their efficacy. In the case of a cleansing composition, the presence of the surfactant complex improves deposition of the skin active agent onto the surface of the skin during the cleansing process. Because the dispersed complex contains a cationic and an anionic surfactant, it is also effective for cleansing the skin and for promoting the desquamation process. Because the charges on the individual surfactants are complexed in the dispersed complex, the surfactants are rendered less harsh and irritating to the skin versus the free surfactants.

The compositions of the present invention can comprise, consist of, or consist essentially of, the essential as well as the optional ingredients components, or limitations described in detail hereinafter.

All parts, percentages, and ratios as used herein are by weight of the total composition, unless otherwise specified. All weight percentages are on an actives weight basis, unless otherwise specified.

The compositions of the present invention can be formulated into a wide variety of product types including, but not limited to, creams, lotions, mousses, sprays, "rinse-off" cleansers, "water-less" cleansers, bars, gels, and the like. The term "rinse-off," as used herein, means that the composition is in a form that can be used in a cleansing process whereby the composition is ultimately rinsed or washed from the skin with water to complete the cleansing process. The term "water-less," as used herein, means that the composition is in a form that can be used in a cleansing process without water, whereby the composition is typically removed by wiping with a device such as a cotton ball, a cotton pad, a tissue, a towel, and the like.

The term "pharmaceutically-acceptable," as used herein, means that the compositions and components thereof are of sufficiently high purity and are suitable for use in contact with human skin and tissues without undue toxicity, irritation, incompatibility, instability, allergic response, and the like.

### Dispersed Surfactant Complex

The topical compositions of the present invention comprise a stable surfactant complex dispersed throughout the composition, and which comprise an anionic surfactant component and a cationic surfactant component. Each of these components is described hereinafter in detail.

Specifically, the surfactant complex comprises from about 25% to about 100% of the cationic surfactant of the topical composition and from about 25% to about 100% of the anionic surfactant of the topical composition.

The dispersed surfactant complex in the topical composition is formed prior to or during formulation of the topical composition, and comprises the anionic and cationic components described herein. Formation of the complex prior to or during formulation is essential for providing the performance benefits described herein. Formation of the requisite complex prior to or during such formulation can be determined by methods known in the art for identifying complex formation such as viscosity and/or conductance measurements.

The surfactant complex in the topical composition herein may also form dispersed coacervates that can be seen by microscopic analysis. Formation and maintenance of such a coacervate system are dependent on a variety of criteria, including component molecular weights, concentrations, ionic strengths, charge density of the cationic and anionic surfactants, pH and temperature. Coacervate systems have been described generally by J. Caelles, et al., "Anionic and Cationic Compounds in Mixed Systems", Cosmetics & Toiletries, Vol. 106, April 1991, pp 49-54; C. J. van Oss, "Coacervation. Complex-Coacervation and Flocculation", J. Dispersion Science and Technology, vol. 9 (5,6) 1988-89, pp561-573; and D.J. Burgess, "Practical Analysis of Complex Coacervate Systems", J. of Colloid and Interace Science, vol. 140, No. 1, November 1990, pp 227-238.

It is believed that the dispersed surfactant complex described herein, whether or not in coacervate form, helps improve deposition of the skin active agents (described hereinafter) onto skin, or otherwise improve performance or efficacy of such skin active agents after such deposition. It is also believed that the dispersed surfactant complex helps provide topical cleansing with improved mildness as compared to similar surfactant systems without the preformed surfactant complex as defined herein. These surfactant complex systems, when incorporated into the topical composition herein, also provide improved skin feel benefits.

Techniques for analysis and detection of anionic/cationic complexes or coacervates are known in the relevant art. For example, microscopic analyses of topical compositions can be used to identify whether a coacervate phase has formed. Such a coacervate phase will be identifiable as an additional emulsified phase in the topical composition. The use of dyes can aid in distinguishing the coacervate phase from the other insoluble phases dispersed in the composition. Components of the surfactant complex or coacervate can also be directly or indirectly identified and quantified by methods known in the relevant art.

### Cationic Surfactant

The topical compositions of the present invention comprise a cationic surfactant in combination with an anionic surfactant (described hereinafter). Known cationic surfactants can be used in the topical composition, provided that such surfactants are compatible with the essential components of the composition, and are pharmaceutically-acceptable for topical application to skin. Concentrations of the cationic surfactant range from about 0.1% to about 20%, more preferably from about 0.2% to about 15%, most preferably from about 0.5% to about 10%, by weight of the composition. As stated hereinbefore, at least some of the cationic surfactant must form a dispersed surfactant complex with the anionic surfactant component prior to or during formulation of the topical composition herein.

Cationic surfactants suitable for use in the composition of the present invention include those described in McCutcheon's, Detergents and Emulsifiers, North American edition (1986), published by allured Publishing Corporation; and McCutcheon's, Functional Materials, North American Edition (1992); It is believed that such cationic materials can also provide an antimicrobial effect to the compositions herein. Therefore, cationic materials having antimicrobial properties are highly useful herein. Cationic surfactants suitable for use in the compositions include cationic ammonium salts such as those represented by the formula 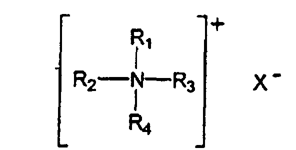 wherein R₁ is an alkyl, aromatic, aryl or alkaryl group having from about 12 to about 22 carbon atoms; R₂, R₃, and R₄ are independently selected from hydrogen, an alkyl group having from about 1 to about 22 carbon atoms, or aromatic, aryl or alkaryl radicals having from about 12 to about 22 carbon atoms; and X is an anion selected from chloride, bromide, iodide, acetate, phosphate, nitrate, sulfate, methyl sulfate, ethyl sulfate, tosylate, lactate, citrate and glycolate. Additionally, the alkyl group can also contain ether linkages, or hydroxy or amino group substituents (e.g., the alkyl groups can contain polyethylene glycol and polypropylene glycol moieties).

More preferably, R₁ is an alkyl group having from about 12 to about 22 carbon atoms; R₂ is selected from H or an alkyl group having from about 1 to about 22 carbon atoms; R₃ and R₄ are independently selected from H or an alkyl group having from about 1 to about 3 carbon atoms; and X is as described in the previous paragraph. Most preferably, R₁ is an alkyl group having from about 12 to about 22 carbon atoms; R₂, R₃, and R₄ are selected from H or an alkyl group having from about 1 to about 3 carbon atoms; and X is as described previously.

Other useful cationic surfactants include amino-amides, wherein in the above structure R₁ is alternatively R₅CO-(CH₂)ₙ -, wherein R₅ is an alkyl group having from about 12 to about 22 carbon atoms, and n is an integer from about 2 to about 6, more preferably from about 2 to about 4, and most preferably from about 2 to about 3. Examples of these cationic emulsifiers include stearamidopropyl PG-dimonium chloride phosphate, stearamidopropyl ethyldimonium ethosulfate, stearamidopropyl dimethyl (myristyl acetate) ammonium chloride, stearamidopropy) dimethyl cetearyl ammonium tosylate, stearamidopropyl dimethyl ammonium chloride, stearamidopropyl dimethyl ammonium lactate, and mixtures thereof.

Examples of quaternary ammonium salt cationic surfactants include cetyl ammonium chloride, cetyl ammonium bromide, lauryl ammonium chloride, lauryl ammonium bromide, stearyl ammonium chloride, stearyl ammonium bromide, cetyl dimethyl ammonium chloride, cetyl dimethyl ammonium bromide, lauryl dimethyl ammonium chloride, lauryl dimethyl ammonium bromide, stearyl dimethyl ammonium chloride, stearyl dimethyl ammonium bromide, cetyl trimethyl ammonium chloride, cetyl trimethyl ammonium bromide, lauryl trimethyl ammonium chloride, lauryl trimethyl ammonium bromide, stearyl trimethyl ammonium chloride, stearyl trimethyl ammonium bromide, lauryl dimethyl ammonium chloride, stearyl dimethyl cetyl ditallow dimethyl ammonium chloride, dicetyl ammonium chloride, dicetyl ammonium bromide, dilauryl ammonium chloride, dilauryl ammonium bromide, distearyl ammonium chloride, distearyl ammonium bromide, dicetyl methyl ammonium chloride, dicetyl methyl ammonium bromide, dilauryl methyl ammonium chloride, dilauryl methyl ammonium bromide, distearyl methyl ammonium chloride, distearyl dimethyl ammonium chloride, distearyl methyl ammonium bromide, and mixtures thereof. Additional quaternary ammonium salts include those wherein the C12 to C22 alkyl carbon chain is derived from a tallow fatty acid or from a coconut fatty acid. The term "tallow" refers to an alkyl group derived from tallow fatty acids (usually hydrogenated tallow fatty acids), which generally have mixtures of alkyl chains in the C 16 to C 18 range. The term "coconut" refers to an alkyl group derived from a coconut fatty acid, which generally have mixtures of alkyl chains in the C12 to C14 range. Examples of quaternary ammonium salts derived from these tallow and coconut sources include ditallow dimethyl ammonium chloride, ditallow dimethyl ammonium methyl sulfate, di(hydrogenated tallow) dimethyl ammonium chloride, di(hydrogenated tallow) dimethyl ammonium acetate, ditallow dipropyl ammonium phosphate, ditallow dimethyl ammonium nitrate, di(coconutalkyl)dimethyl ammonium chloride, di(coconutalkyl)dimethyl ammonium bromide, tallow ammonium chloride, coconut ammonium chforidt, stearamidopropyl PG-dimonium chloride phosphate, stearamidopropyl ethyldimonium ethosulfate, siearamidopropyl dimethyl (myristyl acetate) ammonium chloride, stearamidopropyl dimethyl cetearyl ammonium tosylate. stearamidopropyl dimethyl ammonium chloride, stearamidopropyl dimethyl ammonium lactate, and mixtures thereof.

Preferred cationic surfactants useful herein include dilauryl dimethyl ammoniun chloride, distearyl dimethyl ammonium chloride, dimyristyl dimethyl ammonium chloride, dipalmityl dimethyl ammonium chloride, distearyl dimethyl ammonium chloride, and mixtures thereof.

### Anionic Surfactant

The topical compositions of the present invention comprise an anionic surfactant in combination with the cationic surfactant described hereinbefore. Known anionic surfactants can be used in the topical compositions herein, provided that such surfactants are compatible with the essential components of the compositions and are pharmaceutically-acceptable for topical application to human skin. Concentrations of the anionic surfactant range from about 0.1% to about 20%. more preferably from about 02% to about 10%, and most preferably from about 0.5% to about 5%, by weight of the compositions. As stated hereinbefore, at least some of the anionic surfactant must form a dispersed surfactant complex with the cationic surfactant component prior to or during formulation of the topical composition herein.

Anionic surfactants suitable for use in the compositions of the present invention include those described in McCutcheon's, Detergents and Emulsifiers, North American edition (1986), published by allured Publishing Corporation; McCutcheon's, Functional Materials, North American Edition (1992); and U.S. Patent No. 3,929,678, to Laughlin et al., issued December 30, 1975.

Anionic surfactants suitable for use in the compositions include the alkoyl isethionates, alkyl sulfates and alkyl ether sulfates. The alkoyl isethionates typically have the formula RCO-OCH₂CH₂SO₃M wherein R is alkyl or alkenyl of from about 10 to about 30 carbon atoms, and M is a water-soluble cation such as ammonium, sodium, potassium or triethanolamine. Specific examples of suitable isethionates include ammonium cocoyl isethionate, sodium cocoyl isethionate, sodium lauroyl isethionate, sodium stearoyl isethionate, and mixtures thereof.

Alkyl sulfates and alkyl ether sulfates suitable for use in the compositions are typically those represented by the respective formulae ROSO₃M and RO(C₂H₄O)ₓSO₃M, wherein R is alkyl or alkenyl of from about 10 to about 30 carbon atoms, x is from about 1 to about 10, and M is a water-soluble cation such as ammonium, sodium, potassium or triethanolamine. Another suitable class of anionic surfactants are the water-soluble salts of the organic, sulfuric acid reaction products represented by the formula R₁--SO₃--M
wherein R₁ is chosen from the group consisting of a straight or branched chain, saturated aliphatic hydrocarbon radical having from about 8 to about 24, preferably about 10 to about 16, carbon atoms; and M is a cation. Still other anionic synthetic surfactants include the class designated as succinamates, olefin sulfonates having about 12 to about 24 carbon atoms, and b-alkyloxy alkane sulfonates, examples of which include sodium lauryl sulfate and ammonium lauryl sulfate.

Other suitable anionic surfactants include soaps (i.e. alkali metal salts, e.g., sodium or potassium salts) of fatty acids, typically having from about 8 to about 24 carbon atoms, preferably from about 10 to about 20 carbon atoms. The fatty acids used in making the soaps can be obtained, for example, from natural sources such as plant or animal-derived glycerides (e.g., palm oil, coconut oil, soybean oil, castor oil, tallow, lard, etc.) The fatty acids can also be synthetically prepared.

Other suitable anionic surfactants include phosphates such as monoalkyl, dialkyl, and trialkylphosphate salts, alkanoyl sarcosinates corresponding to the formula RCON(CH₃)CH₂CH₂CO₂M wherein R is alkyl or alkenyl of about 10 to about 20 carbon atoms, and M is a water-soluble cation such as ammonium, sodium, potassium or trialkanolamine (e.g., triethanolamine), preferred examples of which include sodium lauroyl sarcosinate, sodium cocoyl sarcosinate, ammonium lauroyl sarcosinate, and mixtures thereof.

Preferred anionic surfactants for use in the compositions herein include sodium lauryl sulfate, ammonium lauryl sulfate, ammonium cetyl sulfate, sodium cetyl sulfate, sodium stearyl sulfate, ammonium cocoyl isethionate, sodium lauroyl isethionate, sodium lauroyl sarcosinate, and mixtures thereof. Sodium lauryl sulfate is most preferred.

### Skin Active Agents

The topical compositions of the present invention comprise a safe and effective amount of one or more skin active agents or pharmaceutically-acceptable salt or derivative thereof. Concentrations of such skin active agents generally range from about 0.001% to about 20%, preferably from about 0.01% to about 15%, more preferably from about 0.025% to about 10%, by weight of the topical compositions.

As used herein, "skin active agents" are pharmaceutially-acceptable materials for application onto human skin, and which provide anti-acne activity, anti-wrinkling activity, topical anesthetic activity, topical antimicrobial activity, topical anti-inflammatory activity, or combinations thereof, upon topical application onto human skin.

The phrase "safe and effective amount" as used herein, refers to the concentration of an active ingredient sufficiently high to modify the condition to be treated or to deliver the desired skin benefit, but sufficiently low to avoid serious side effects, at a reasonable benefit to risk ratio within the scope of sound medical judgment. Safe and effective amounts as used in this context may vary with each specific skin active agent.

Suitable skin active agents which provide anti-acne and/or anti-wrinkling activity to human skin, include keratolytics (salicylic acid, derivatives of salicylic acid, alpha-hydroxy acids (glycolic acid, phytic acid, lactic acid, phytic acid, lipoic acid; lysophosphatidic acid), retinoids (retinol. retinoic acid, retinal and retinyl esters such as retinyl acetate, retinyl butyrate, retinyl propionate, retinyl octanoate, retinyl laurate, retinyl palmitate, retinyl oleate and retinyl linoleate), bioflavanoids, niacinamide, N-acetylcysteine (including N-acetyl-L-cysteine, N-acetyl-D-cystcine, pharmaceutically acceptable derivatives or salts thereof), and combinations thereof.

Suitable skin active agents which provide topical antimicrobial activity (including antibacterial and antifungal activity), or topical antimicrobial and anti-acne activity, are benzoyl peroxide, octopirox, tetracycline, 2,4,4'-trichloro-2'-hydroxy diphenyl ether, 3,4,4'-trichlorobanilide, azelaic acid and its derivatives, phenoxyethanol, phenoxypropanol, phenoxyisopropanol, ethyl acetate, clindamycin, erythromycin, metronidazole, ketoconazole, and meclocycline.

Suitable skin active agents which provide topical anti-inflammatory activity include topical anti-inflammatory steroids, and topical non-steroidal anti-inflammatory agents. Examples of such suitable agents include acetyl salicylic acid, ibuprofen, naproxen, benoxaprofen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen and bucloxic acid. Other suitable anti-inflammatory agents are described, for example, in U.S. Patent 4,985,459. Suitable steroidal anti-inflammatory agents are those well known in the pharmaceutical art for use in topical compositions.

Suitable skin active agents which provide topical anesthetic activity include benzocaine, lidocaine, bupivacaine, chlorprocaine, dibucaine, etidocaine, mepivacaine, tetracaine, dyclonine, hexylcaine, procaine, cocaine, ketamine, pramoxine, phenol, and pharmaceutically acceptable salts thereof.

### Water

The topical compositions of the present invention comprise from about 40% to about 99%, more preferably from about 60% to about 95%, and most preferably from about 70% to about 90% , by weight of water. Specific water concentrations will depend upon the product form selected and the desired moisture content.

### Optional Components

The topical compositions of the present invention may further comprise one or more optional components, provided that such optional components are pharmaceutically-acceptable for topical application onto human skin, and are compatible with the essential components of the topical compositions.

Examples of suitable optional components are described in the CTFA Cosmetic Ingredient Handbook, Second Edition, 1992. Examples suitable optional materials include absorbents, abrasives, anticaking agents, antifoaming agents, antioxidants, binders, biological additives, buffering agents, bulking agents, chelating agents, chemical additives, colorants, cosmetic astringents, cosmetic biocides, denaturants, drug astringents, emulsifiers, essential oils, skin sensates, skin soothing agents, skin healing agents, film formers, fragrance components, humectants, opacifying agents, pigments, pH adjusters, plasticizers, preservatives, propellants, reducing agents, skin bleaching agents, skin-conditioning agents (emollient, humectants, miscellaneous, and occlusive), skin protectants, solvents, foam boosters, hydrotropes, solubilizing agents, suspending agents (nonsurfactant), sunscreen agents, ultraviolet light absorbers, vitamins and vitamin derivatives, viscosity increasing agents (aqueous and nonaqueous), emulsifiers, sequestrants, skin sensates, and combinations thereof.

Other optional components are described in detail hereinafter.

### a) Optional Surfactants

The topical compositions of the present invention, in addition to the required anionic and cationic surfactant components, may further comprise additional surfactants, provided that such additional surfactants are pharmaceutially acceptable for application on skin, and are compatible with the essential components of the compositions herein.

Optional nonionic surfactants are preferred, concentrations of which may range from about 0.1% to about 15%, more preferably from about 0.2% to about 10%, and most preferably from about 0.5% to about 5%, by weight of the compositions. Examples of suitable nonionic surfactants are described in McCutcheon's, Detergents and Emulsifiers, North American edition (1986), published by allured Publishing Corporation; and McCuteheon's, Functional Materials, North American Edition (1992).

Optional amphoteric surfactants may also be used in the compositions herein, concentrations of which can range from about 0.1% to about 20%. more preferably from about 0.2% to about 10%, most preferably from about 0.5% to about 5%, by weight of the compositions. The optional amphoteric surfactants suitable for use herein includes zwitterionic surfactants, which are well known to formulators skilled in the art as a subset of amphoteric surfactants. Examples of suitable amphoteric surfactants are described in McCuteheon's, Detertents and Emulsifiers, North American edition (1986), published by Allured Publishing Corporation; and McCuteheon's, Functional Materials, North American Edition (1992);

### b) Humectants and Moisturizers

The topical compositions of the present invention may further comprise one or more humectants or moisturizers. Any known humectant or moisturizer may be used, provided that it is pharmaceutially acceptable for application to skin, and is compatible with the essential components of the compositions herein. Concentrations of such materials can range from about 0.1% to about 20%, more preferably from about 0.5% to about 10%, most preferably from about 1% to about 5%, by weight of the compositions

Humectants suitable for use herein include, but are not limited to, guanidine; glycolic acid and glycolate salts (e.g., ammonium and quaternary alkyl ammonium); lactic acid and lactate salts (e.g., ammonium and quaternary alkyl ammonium); aloe vera in any of its variety of forms (e.g., aloe vera gel); polyhydroxy alcohols such as sorbitol, glycerol, hexanetriol, propylene glycol, butylene glycol, hexylene glycol and the like; polyethylene glycols; sugars and starches; sugar and starch dtrivatives (e.g., alkoxylated glucose); hyaluronic acid; lactamide monoethanolamine; acetamide manoethanolamine; propoxylated glyercerols as described in U.S. Patent No. 4,976,953; and combinations thereof. Glycerol is the preferred humectant.

### c) Emulsifiers

The topical compositions of the present invention may further comprise one or more emulsifiers for emulsifying the various carrier components of the compositions. Concentrations of the emulsifiers can range from 0.1% to about 10%, more preferably from about 1% to about 7%, most preferably from about 1% to about 5%, by weight of compositions.

Any known emulsifier can be used in the composition, provided that it is pharmaceutically-acceptable for application to skin, and is compatible with the essential components of the compositions. Examples of suitable emulsifiers include nonionic, cationic, anionic, and zwitterionic emulsifiers described in McCutcheon's, Determents and Emulsifiers, North American Edition (1986), published by Allured Publishing Corporation; U.S. Patent No. 5,011,681; U.S. Patent No. 4,421,769; and U.S. Patent No. 3,755,560;

Suitable emulsifiers include esters of glycerin, esters of propylene glycol, fatty acid esters of polyethylene glycol, fatty acid esters of polypropylene glycol, esters of sorbitol, esters of sorbitan anhydrides, carboxylic acid copolymers, esters and ethers of glucose, ethoxylated ethers, ethoxylated alcohols, alkyl phosphates, polyoxyethylene fatty ether phosphates, fatty acid amides, acyl lactylates, soaps and mixtures thereof.

### d) Sunscreens and Tanning Agents

The compositions of the present invention may further comprise one or more pharmaceutically-acceptable sunscreens known in the art, provided that such sunscreen materials are compatible with the essential components of the compositions. Sunscreening agents are described, for example, in U.S. Patent No. 5,087,445; U.S. Patent No. 5,073,372; U.S. Patent No. 5,073,371; and Segarin, et al., at Chapter VIII. pages 189 et seq., of Cosmetics Science and Technology.

Concentrations of the sunscreens can range from about 0.5% to about 20% by weight of the compositions. Exact amounts will vary depending upon the selected sunscreen and the Sun Protection Factor (SPF) desired for the composition.

The topical compositions of the present invention may further comprise one or more artificial tanning agent or accelerator, examples of which include dihydroxyacetone, tyrosine, tyrosine esters such as ethyl tyrosinate, and phospho-DOPA.

### e) Insoluble Particles

The topical compositions of the present invention may further comprise from about 0.1% to about 20%, preferably from about 0.25% to about 15%, most preferably from about 0.5% to about 10%, of insoluble particles by weight of the compositions, which insoluble particles are useful for enhancing the cleansing effect when the topical compositions are in the form of a cleansing composition.

The term "insoluble" as used in this context means that the particles are essentially insoluble in the compositions of the present invention. In particular, the insoluble particles should have a solubility less than about 1 gram per 100 grams of composition at 25°C. preferably less than about 0.5 grams per 100 grams of composition at 25°C, and more preferably less than about 0.1 grams per 100 grams of composition at 25°C.

The insoluble particles may be micronized or of conventional size. The micronized particles, for the most part, are of a size that is below the tactile threshold and are essentially nonabrasive to the skin. The conventional size particles are tactilely perceptible and are added for the scrubbing and abrasive effect which they provide.

The micronized particles have a mean particle size diameter and particle size distribution such that they are below the tactile perception threshold of most users, and yet are not so small as to be ineffective for aiding in oil, dirt, debris, and make-up removal. It has been found that particles having a mean particle size diameter greater than about 75 microns are tactilely perceived during the cleansing process and it is important to minimize the amount of these larger particles if it is desired that the particles not be felt by the user. Conversely, it is found that particles having a mean particle size diameter of less than about 1 to about 5 microns are generally less effective for providing a cleansing benefit. Without being limited by theory, it is believed that the micronized cleansing particles should be of a size that is on the order of the thickness of the dirt, oil, or debris layer to be cleaned away. This layer is believed to be on the order of a few microns in thickness in most instances. Particles having a wide range of shapes, surface characteristics, and hardness characteristics may be utilized herein.

Micronized particles of the present invention can be derived from a wide variety of materials including those derived from inorganic, organic, natural, and synthetic sources. Nonlimiting examples of these materials include those selected from the group consisting of almond meal, alumina, aluminum oxide, aluminum silicate, apricot seed powder, attapulgite, barley flour, bismuth oxychloride, boron nitride, calcium carbonate, calicum phosphate, calcium pyrophosphate, calicum sulfate, cellulose, chalk, chitin, clay, corn cob meal, corn cob powder, corn flour, corn meal, corn starch, diatomaceous earth, dicalcium phosphate, dicalcium phosphate dihydrate, fullers earth, hydrated silica, hydroxyapatite, iron oxide, jojoba seed powder, kaolin, loofah, magneisum trisilicate, mica, microcrystalline cellulose, montmorillonite, oat bran, oat flour, oatmeal, peach pit powder, pecan shell powder, polybutylene, polyethylene, polyisobutylene, polymethylstyrene, polypropylene, polystyrene, polyurethane, nylon, teflon (i.e. polytetrafluoroethylene), polyhalogenated olefins, pumice rice bran, rye flour, sericite, silica, silk, sodium bicarbonate, soidum silicoaluminate, soy flour synthetic hectorite, talc, tin oxide, titanium dioxide, tricalcium phosphate, walnut shell powder, wheat bran, wheat flour, wheat starch, zirconium silicate, and mixtures thereof. Also useful are micronized particles made from mixed polymers (e.g., copolymers, terpolymers, etc.), such as polyethylene/polypropylene copolymer, polyethylene/propylene/isobutylene copolymer, polyethylene/styrene copolymer, and the like. Typically, the polymeric and mixed polymeric particles are treated via an oxidation process to destroy impurities and the like. The polymeric and mixed polymeric particles can also optionally be crosslinked with a variety of common crosslinking agents, nonlimiting examples of which include butadiene, divinyl benzene, methylenebisacrylamide, allyl ethers of suscrose, allyl ethers of pentaerythritol, and mixtures thereof. Other examples of useful micronized particles include waxes and resins such as paraffins, carnuba wax, ozekerite wax, candellila wax, urea-formaldehyde resins, and the like. When such waxes and resins are used herein it is important that these materials are solids at ambient and skin temperatures.

Among the preferred water-insoluble, micronized particulate materials useful herein are the synthetic polymeric particles selected from the group consisting of polybutylene, polyethylene, polyisobutylene, polymethylstyrene, polypropylene, polystyrene, polyurethane, nylon, teflon, and mixtures thereof. Most preferred are polyethylene and polypropylene micronized particles, with the oxidized versions of these materials being especially preferred. Examples of commercially available particles useful herein include the ACumist™ micronized polyethylene waxes available from Allied Signal (Morristown, NJ) available as the A, B, C, and D series in a variety of average particle sizes ranging from 5 microns to 60 microns. Preferred are the ACumist™ A-25, A-30, and A-45 oxidized polyethylene particles having a mean particle size of 25, 30, and 45 microns, respectively. Examples of commercially available polypropylene particles include the Propyltex series available from Micro Powders (Dartek).

The conventional size insoluble particles are well-known to formulation chemists in the art. These particles typically have larger particle sizes than the micronized particles described herein. These particles generally have an average particle size diameter that is about 75 microns or greater, which is about the tactile threshold described above. These conventional size particles typically have average particle sizes ranging up to about 400 microns and larger. These particles can be made from the same materials as for the micronized particles just described. Among the preferred conventional size particulate materials useful herein are the synthetic polymeric particles selected from the group consisting of polybutylene, polyethylene, polyisobutylene, polymethylstyrene, polypropylene, polystyrene, polyurethane, nylon, teflon, and mixtures thereof. Most preferred are polyethylene and polypropylene micronized particles, with the oxidized versions of these materials being especially preferred. An example of a commercially available conventional size particle useful herein is ACuscrub™ 51, available from Allied Signal (Morristown, NJ) having a mean particle size of about 125 microns.

### f) Oils

The topical compositions of the present invention may further comprise one or more oils. Any known oil may be used, provided that it is pharmaceutically acceptable for application to the skin, and is compatible with the essential components of the compositions herein. Concentrations of such materials can range from about 0.25% to about 40%, preferably from about 0.5% to about 25%, and more preferably from about 0.75% to about 15%, by weight of the composition.

Oils generally have low solubility in water, generally less than about 1% by weight at 25°C. Examples of oils suitable for use herein include mineral oil, petrolatum, C7-C40 branched chain hydrocarbons, C1-C30 alcohol esters of C1-C30 carboxylic acids, C1-C30 alcohol esters of C2-C30 dicarboxylic acids, monoglycerides of C1-C30 carboxylic acids, diglycerides of C1-C30 carboxylic acids, triglycerides of C1-C30 carboxylic acids, ethylene glycol monoesters of C1-C30 carboxylic acids, ethylene glycol diesters of C1-C30 carboxylic acids, propylene glycol monoesters of C1-C30 carboxylic acids, propylene glycol diesters of C1-C30 carboxylic acids, C1-C30 carboxylic acid monoesters and polyesters of sugars, polydialkylsiloxanes, polydiarylsiloxanes, polyalkarylsiloxanes, cylcomethicones having 3 to 9 silicon atoms, vegetable oils, hydrogenated vegetable oils, polypropylene glycols. polypropylene glycol C4-C20 alkyl ethers, di C8-C30 alkyl ethers, and mixtures thereof. Some of these oils are further described in U.S. Patent 4,919,934, to Deckner et al., issued April 24 1990, which descriptions are incorporated herein by reference.

### METHOD OF FORMING THE COMPLEX

The dispersed surfactant complex of the topical compositions herein must be formed prior to or during formulation of the topical compositions. Preferably, the dispersed complex is preformed by initially combining some or all of the anionic surfactant component, with some or all of the cationic surfactant component, in an aqueous solution, to thereby form an aqueous dispersion of the preformed anionic/cationic complex. The aqueous dispersion is then combined with the skin active agent and any remaining components.

### METHODS OF TREATING THE SKIN

The present invention is also directed to methods wherein an effective amount of the topical composition of the present invention is applied to the skin. These topical compositions are useful for delivering skin active agents to the skin. A wide range of quantities of the topical compositions may be applied to the skin, but will typically range from about 0.1 mg/cm² to about 25 mg/cm².

In preferred embodiments, the compositions of the present invention are also useful for personal cleansing, especially for cleansing of the face and neck areas. Typically, suitable amount of the cleansing composition is applied directly to the area to be cleansed, or indirectly applied by using a washcloth, sponge, pad, cotton ball or other indirect application means. If desired, the area to be cleansed can be premoistened with water.

It has been found that the topical compositions of the present invention can be combined with water during the cleansing process and rinsed-off from the skin. Alternatively, the composition can be used alone and wiped-off from the skin using a pad, cotton ball, tissue, or other like device. The cleansing process is typically a two-step process involving application of the composition followed either by rinsing of the product with water or wiping without the use of water. Generally, an effective amount of composition to be used will depend upon the needs and usage habits of the individual.

### EXAMPLES

The compositions illustrated in the following examples illustrate specific embodiments of the topical compositions of the present invention, but are not intended to be limiting thereof. Each of the exemplified embodiments of the present invention provide excellent cleansing of the skin and improved deposition of the skin active agent therein.

All exemplified compositions can be prepared by conventional formulation and mixing techniques. Component amounts are listed as weight percents and exclude minor materials such as diluents, filler, and so forth. The listed formulations, therefore, comprise the listed components and any minor materials associated with such components.

### Example 1

A personal cleansing composition containing salicylic acid is prepared by combining the following ingredients using conventional mixing techniques.

| Ingredients | Weight Percent |
|---|---|
| Phase A | |
| Water | QS 100 |
| Glycerin | 3.00 |
| Tetrasodium EDTA | 0.02 |

| Phase B | |
|---|---|
| PPG-15 Stearyl Ether | 4.00 |
| Stearyl Alcohol | 0.75 |
| Salicylic Acid | 2.00 |
| Cetyl Alcohol | 0.75 |
| Steareth-21 | 0.45 |
| Steareth-2 | 0.05 |
| Dimethicone | 0.60 |
| Polyquaternium-37 (and) Mineral Oil (and) PPG-1 Trideceth-6 | 1.50 |

| Phase C | |
|---|---|
| Triethanolamine | 0.15 |

| Phase D | |
|---|---|
| Fragrance | 0.10 |

| Phase E | |
|---|---|
| PG-Dimonium Chloride Phosphate | 2.00 |
| Sodium Lauryl Sulfate | 1.00 |

In a suitable vessel, the Phase A ingredients are heated with stirring to about 75°C. In a separate vessel, the Phase B ingredients are heated with stirring to about 75°C. Phase B is then added to Phase A with mixing, followed by the addition of Phase C, with mixing. The fragrance is then added with continued mixing. The mixture is cooled to 35°C. In a separate vessel, the Phase E ingredients are combined and added to the remaining mixture with stirring.

The resulting leave-on topical composition is useful for clarifying the skin, and in particular for preventing and treating acne while being mild to the skin. Alternatively, a composition is prepared in which the sodium lauryl sulfate is replaced with sodium lauroyl isetheonate.

### Example 2

A personal cleansing composition containing salicylic acid is prepared by combining the following ingredients using conventional mixing techniques.

| Ingredients | Weight Percent |
|---|---|
| Phase A | |
| Water | QS 100 |
| Glycerin | 3.00 |
| Disodium EDTA | 0.01 |

| Phase B | |
|---|---|
| PPG-15 Stearyl Ether | 4.00 |
| Stearyl Alcohol | 2.88 |
| Salicylic Acid | 2.00 |
| Cetyl Alcohol | 0.80 |
| Steareth-21 | 0.50 |
| Behenyl Alcohol | 0.32 |
| PPG-30 | 0.25 |
| Steareth-2 | 0.25 |

| Phase C | |
|---|---|
| Oxidized Polyethylene Beads¹ | 1.00 |
| Fragrance | 0.27 |

| Phase D | |
|---|---|
| Distearyl Dimethyl Ammonium Chloride | 2.00 |
| Sodium Lauryl Sulfate | 1.00 |

| | |
|---|---|
| ¹Available as Acucscrub™ 51 from Allied Signal Corporation. | |

In a suitable vessel, the Phase A ingredients are heated with stirring to about 75°C. In a separate vessel, the Phase B ingredients are heated with stirring to about 75°C. Phase B is then added to Phase A with mixing. Next, the oxidized polyethylene beads are added slowly with mixing to prevent agglomeration. The fragrance is then added with continued mixing. The mixture is cooled to 35°C. In a separate vessel, the Phase D ingredients are combined and added to the remaining mixture with stirring.

The resulting cleansing composition is useful for cleansing the skin.

Alternatively, a composition is prepared in which the sodium lauryl sulfate is replaced with sodium lauroyl isetheonate.

Alternatively, a composition is prepared in which the salicylic acid is replaced with 2.5% benzoyl peroxide, with the water being adjusted accordingly.

### Example 3

A personal cleansing composition containing salicylic acid and menthol is prepared by combining the following ingredients using conventional mixing techniques.

| Ingredients | Weight Percent |
|---|---|
| Phase A | |
| Water | QS 100 |
| Glycerin | 3.00 |
| Disodium EDTA | 0.01 |

| Phase B | |
|---|---|
| PPG-15 Soeatyl Ether | 4.00 |
| Stearyl Alcohol | 2.88 |
| Salicylic Acid | 2.00 |
| Cetyl Alcohol | 0.80 |
| Steareth-21 | 0.50 |
| Behenyl Alcohol | 0.32 |
| PPG-30 | 0.25 |
| Steareth-2 | 0.25 |

| Phase C | |
|---|---|
| Oxidized Polyethylene Beads¹ | 1.00 |
| Fragrance | 0.27 |
| Menthol | 0.05 |

| Phase D | |
|---|---|
| Distearyl Dimethyl Ammonium Chloride | 2.00 |
| Sodium Lauryl Sulfate | 1.00 |

| | |
|---|---|
| ¹Available as Acucscrub™ 51 from Allied Signal Corporation. | |

In a suitable vessel, the Phase A ingredients are heated with stirring to about 75°C. In a separate vessel, the Phase B ingredients are heated with stirring to about 75°C. Phase B is then added to Phase A with mixing. Next, the oxidized polyethylene beads are added slowly with mixing to prevent agglomeration. The fragrance and menthol are then added with continued mixing. The mixture is cooled to 35°C. In a separate vessel, the Phase D ingredients are combined and added to the remaining mixture with stirring.

The resulting topical cleansing composition is useful for cleansing the skin. Alternatively, a composition is prepared in which the sodium lauryl sulfate is replaced with sodium lauroyl isetheonate. Alternatively, a composition is prepared in which the salicylic acid is replaced with 2.5% benzoyl peroxide with the water being adjusted correspondingly.

### Example 4

A leave-on lotion composition containing benzoyl peroxide is prepared by combining the following ingredients using conventional mixing techniques.

| Ingredients | Weight Percent |
|---|---|
| Phase A | |
| Water | QS 100 |
| Glycerin | 4.00 |
| Disodium EDTA | 0.10 |
| Carbomer | 0.60 |
| Acrylates/C10-30 Alkylacrylates Crosspolymer | 0.05 |

| Phase B | |
|---|---|
| Stearyl Alcohol | 2.25 |
| Cetyl Alcohol | 2.25 |
| Steareth-100 | 0.50 |
| Glyceryl Hydroxystearate | 0.74 |
| Dimethicone | 0.60 |

| Phase C | |
|---|---|
| Triethanolamine | 0.50 |

| Phase D | |
|---|---|
| Benzoyl Peroxide | 2.50 |

| Phase E | |
|---|---|
| Dicetyl Dimethyl Ammonium Chloride | 1.00 |
| Sodium Lauryl Sulfate | 0.50 |

In a suitable vessel, the Phase A ingredients are heated with stirring to about 75°C. In a separate vessel, the Phase B ingredients are heated with stirring to about 75°C. Phase B is then added to Phase A with mixing. Next Phase C is added with mixing. Next, the mixture is cooled to 35°C. Next the benzoyl peroxide is added with mixing. In a separate vessel, the Phase E ingredients are combined and added to the remaining mixture with stirring.

The resulting topical leave-on composition is useful for clarifying the skin, and in particular for preventing and treating acne while being mild to the skin.

### Examples 5

A personal cleansing gel composition containing glycolic acid is prepared by combining the following ingredients using conventional mixing techniques.

| Ingredients | Weight Percent |
|---|---|
| Phase A | |
| Water | QS 100 |
| Glycerin | 4.00 |
| Disodium EDTA | 0.10 |
| Dimethicone | 0.20 |
| PVM/MA Decadiene Crosspolymer | 1.00 |
| Glycolic Acid | 2.00 |
| Sodium Hydroxide | 0.80 |

| Phase B | |
|---|---|
| Ammonium chloride | 1.00 |
| Sodium Lauryl Sulfate | 0.50 |

In a suitable vessel, the Phase A ingredients are mixed vigorously. In a separate vessel, the Phase B ingredients are combined and added to the remaining mixture with stirring.

The resulting cleansing gel composition is useful for providing skin clarification, and in particular for reducing the appearance of pore size, while being mild to the skin and cleansing effectively. Alternatively, a composition is prepared in which glycolic acid is replaced with salicylic acid and the resulting composition is useful for preventing and treating acne and blemishes while being mild to the skin and cleansing effectively.

## Claims

1. A topical composition for delivery of skin active agents to the skin, which composition comprises:
(a) from 0.1% to 20% by weight of a cationic surfactant, ;
(b) from 0.1% to 20% by weight of an anionic surfactant;
(c) from 0.001% to 20% by weight of a skin active agent;
(d) from 40% to 99% by weight of water; and
(e) dispersed surfactant complex formed prior to or during formulation of the topical composition, which complex comprises from 25% to 100%, preferably 100%, of the cationic surfactant of the composition and from 25% to 100%, preferably 100%, of the anionic surfactant of the composition, wherein the net cationic charge density of the combination of cationic and anionic surfactants in the composition is zero.

2. The composition of Claim 1 wherein the skin active agent is selected from the group consisting of anti-acne actives, anti-wrinkle and skin atrophy actives, antimicrobial actives, anti-inflammatory actives, topical anesthetic agents, and combinations thereof, and wherein the cationic surfactant is selected from the group consisting of dilauryl dimethyl ammoniun chloride, distearyl dimethyl ammonium chloride, dimyristyl dimethyl ammonium chloride, dipalmityl dimethyl ammonium chloride and mixtures thereof, and wherein the anionic surfactant is selected from the group consisting of alkyl sulfates, alkyl ether sulfates, alkoyl isethionates, alkanoyl sarcosinates, alkylphosphates and mixtures thereof.

3. The composition of Claim 2 wherein the cationic surfactant has the formula: 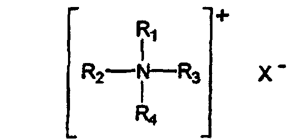 wherein R₁ is an alkyl, aromatic, aryl or alkaryl group having from 12 to 22 carbon atoms; R₂, R₃ and R₄ are independently H or an alkyl group having from 1 to 22 carbon atoms; and X is chloride, bromide, iodide, acetate, phosphate, nitrate, sulfate, methyl sulfate, ethyl sulfate, tosylate, lactate, citrate or glycolate.

4. The composition of Claim 3 wherein the R₁ is an alkyl group having from 12 to 22 carbon atoms, R₂ is H or an alkyl group having from 1 to 22 carbon atoms, R₃ and R₄ are independently H or an alkyl group having from 1 to 3 carbon atoms.

5. The composition of Claim 2 wherein the anionic surfactant is selected from the group consisting of sodium lauryl sulfate, ammonium lauryl sulfate, ammonium cetyl sulfate, sodium cetyl sulfate, sodium stearyl sulfate, ammonium cocoyl isethionate, sodium lauroyl isethionate, sodium lauroyl sarcosinate, and mixtures thereof.

6. The composition of Claim 2 wherein said composition further comprises from 0.1% to 20% by weight of a humectant wherein said humectant is glycerol.

7. A non-therapeutic method for treating skin with skin active agents, which method comprises the step of applying to the skin from 0.5 mg/cm² to 25 mg/cm² of the composition of any one of the preceding claims.

8. A method for cleansing skin, which method comprises the steps of:
(i) applying to the skin from 0.5 mg/cm² to 25 mg/cm² of the composition of any one of the preceding Claims 1, 2, 3, 4, 5 or 6, and
(ii) rinsing or wiping the composition from the skin.

9. A method for clarifying skin and reducing pore size, which method comprises the step of applying to the skin from 0.5 mg/cm² to 25 mg/cm² of the composition of any one of the preceding Claims 1, 2, 3, 4, 5, or 6.

## Patentansprüche

1. Topische Zusammensetzung für die Abgabe hautaktiver Mittel an die Haut, wobei die Zusammensetzung umfaßt:
(a) 0,1 bis 20 Gew.-% eines kationischen Tensids;
(b) 0,1 bis 20 Gew.-% eines anionischen Tensids;
(c) 0,001 bis 20 Gew.-% eines hautaktiven Mittels;
(d) 40 bis 99 Gew.-% Wasser; und
(e) einen dispergierten Tensidkomplex, gebildet vor oder während der Formulierung der topischen Zusammensetzung, wobei der Komplex 25% bis 100%, vorzugsweise 100% des kationischen Tensids der Zusammensetzung und 25% bis 100%, vorzugsweise 100% des anionischen Tensids der Zusammensetzung umfaßt, wobei die kationische Netto-Ladungsdichte der Kombination aus kationischen und anionischen Tensiden in der Zusammensetzung null ist.

2. Zusammensetzung nach Anspruch 1, wobei das hautaktive Mittel ausgewählt ist aus der Gruppe, bestehend aus Anti-Aknemitteln, Anti-Falten- und Hautatrophie-Mitteln, antimikrobiellen Mitteln, entzündungshemmenden Mitteln, topischen anästhetischen Mitteln und Kombinationen davon, und wobei das kationische Tensid ausgewählt ist aus der Gruppe, bestehend aus Dilauryldimethylammoniumchlorid, Distearyldimethylammoniumchlorid, Dimyristyldimethylammoniumchlorid, Dipalmityldimethylammoniumchlorid und Mischungen davon, und wobei das anionische Tensid ausgewählt ist aus der Gruppe, bestehend aus Alkylsulfaten, Alkylethersulfaten, Alkoylisethionaten, Alkanoylsarcosinaten, Alkylphosphaten und Mischungen davon.

3. Zusammensetzung nach Anspruch 2, wobei das kationische Tensid die Formel besitzt: 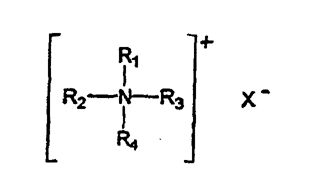 worin R₁ eine Alkyl-, aromatische, Aryl- oder Alkaryl-Gruppe mit 12 bis 22 Kohlenstoffatomen ist; R₂, R₃ und R₄ unabhängig voneinander H oder eine Alkylgruppe mit 1 bis 22 Kohlenstoffatomen sind; und X Chlorid, Bromid, Iodid, Acetat, Phosphat, Nitrat, Sulfat, Methylsulfat, Ethylsulfat, Tosylat, Lactat, Citrat oder Glycolat ist.

4. Zusammensetzung nach Anspruch 3, worin R₁ eine Alkylgruppe mit 12 bis 22 Kohlenstoffatomen ist, R₂ H oder eine Alkylgruppe mit 1 bis 22 Kohlenstoffatomen ist, R₃ und R₄ unabhängig voneinander H oder eine Alkylgruppe mit 1 bis 3 Kohlenstoffatomen sind.

5. Zusammensetzung nach Anspruch 2, wobei das anionische Tensid ausgewählt ist aus der Gruppe, bestehend aus Natriumlaurylsulfat, Ammoniumlaurylsulfat, Ammoniumcetylsulfat, Natriumcetylsulfat, Natriumstearylsulfat, Ammoniumcocoyiisethionat, Natriumlauroylisethionat, Natriumlauroylsarcosinat und Mischungen davon.

6. Zusammensetzung nach Anspruch 2, wobei die Zusammensetzung weiterhin 0,1 bis 20 Gew.-% eines Feuchthaltemittels umfaßt, wobei das Feuchthaltemittel Glycerin ist.

7. Nicht-therapeutisches Verfahren zur Behandlung der Haut mit hautaktiven Mitteln, wobei das Verfahren den Schritt des Auftragens von 0,5 mg/cm² bis 25 mg/cm² der Zusammensetzung nach irgendeinem der vorangehenden Ansprüche auf die Haut umfaßt.

8. Verfahren zum Reinigen der Haut, wobei das Verfahren die Schritte umfaßt:
(i) Auftragen von 0,5 mg/cm² bis 25 mg/cm² der Zusammensetzung nach irgendeinem der vorangehenden Ansprüche 1, 2, 3, 4, 5 oder 6 auf die Haut, und
(ii) Abwaschen oder Abwischen der Zusammensetzung von der Haut.

9. Verfahren zum Klären der Haut und Reduzieren der Porengröße, wobei das Verfahren die Schritte des Auftragens von 0,5 mg/cm² bis 25 mg/cm² der Zusammensetzung nach irgendeinem der vorangehenden Ansprüche 1, 2, 3, 4, 5 oder 6 auf die Haut umfaßt.

## Revendications

1. Composition topique pour diffuser des agents actifs cutanés sur la peau, cette composition comprenant :
(a) de 0,1 % à 20 % en poids d'un agent tensioactif cationique ;
(b) de 0,1 % à 20 % en poids d'un agent tensioactif anionique ;
(c) de 0,001 % à 20 % en poids d'un agent actif cutané ;
(d) de 40 % à 99 % en poids d'eau ; et
(e) un complexe tensioactif dispersé formé avant ou pendant la formulation de la composition topique, ce complexe comprenant de 25 % à 100 %, de préférence 100 %, de l'agent tensioactif cationique de la composition et de 25 % à 100 %, de préférence 100 %, de l'agent tensioactif anionique de la composition, la densité de charge cationique nette de la combinaison d'agents tensioactifs cationique et anionique dans la composition étant de zéro.

2. Composition selon la revendication 1, dans laquelle l'agent actif cutané est choisi dans le groupe constitué par les substances actives anti-acnéiques, les substances actives antirides et contre l'atrophie cutanée, les substances actives antimicrobiennes, les substances actives anti-inflammatoires, les agents anesthésiques locaux et leurs combinaisons, et dans laquelle l'agent tensioactif cationique est choisi dans le groupe constitué par le chlorure de dilauryl diméthyl ammonium, le chlorure de distéaryl diméthyl ammonium, le chlorure de dimyristyl diméthyl ammonium, le chlorure de dipalmityl diméthyl ammonium et leurs mélanges, et dans laquelle l'agent tensioactif anionique est choisi dans le groupe constitué par les sulfates d'alkyle, les éther sulfates d'alkyle, les iséthionates d'alcoyle, les sarcosinates d'alcanoyle, les phosphates d'alkyle et leurs mélanges.

3. Composition selon la revendication 2, dans laquelle l'agent tensioactif cationique a la formule : 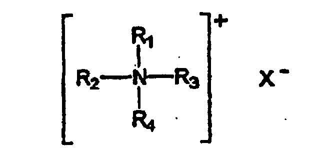 dans laquelle R₁ est un groupe alkyle, aromatique, aryle ou alcaryle ayant de 12 à 22 atomes de carbone ; R₂, R₃ et R₄ sont indépendamment H ou un groupe alkyle ayant de 1 à 22 atomes de carbone ; et X est un chlorure, bromure, iodure, acétate, phosphate, nitrate, sulfate, le sulfate de méthyle, le sulfate d'éthyle, un tosylate, lactate, citrate ou glycolate.

4. Composition selon la revendication 3, dans laquelle R₁ est un groupe alkyle ayant de 12 à 22 atomes de carbone, R₂ est H ou un groupe alkyle ayant de 1 à 22 atomes de carbone, R₃ et R₄ sont indépendamment H ou un groupe alkyle ayant de 1 à 3 atomes de carbone.

5. Composition selon la revendication 2, dans laquelle l'agent tensioactif anionique est choisi dans le groupe constitué par le laurylsulfate de sodium, le laurylsulfate d'ammonium, le cétylsulfate d'ammonium, le cétylsulfate de sodium, le stéarylsulfate de sodium, le cocoyliséthionate d'ammonium, le lauryliséthionate de sodium, le lauroylsarcosinate de sodium et leurs mélanges.

6. Composition selon la revendication 2, ladite composition comprenant en outre de 0,1 % à 20 % en poids d'un humectant, ledit humectant étant le glycérol.

7. Procédé non thérapeutique pour traiter la peau avec des agents actifs cutanés, ce procédé comprenant l'étape consistant à appliquer sur la peau de 0,5 mg/cm² à 25 mg/cm² de la composition selon l'une quelconque des revendications précédentes.

8. Procédé pour nettoyer la peau, ce procédé comprenant les étapes consistant :
(i) à appliquer sur la peau de 0,5 mg/cm² à 25 mg/cm² de la composition selon l'une quelconque des revendications 1, 2, 3, 4, 5 ou 6 précédentes, et
(ii) à rincer ou essuyer la composition de la peau.

9. Procédé pour clarifier la peau et réduire la taille des pores, ce procédé comprenant l'étape consistant à appliquer sur la peau de 0,5 mg/cm² à 25 mg/cm² de la composition selon l'une quelconque des revendications 1, 2, 3, 4, 5 ou 6 précédentes.
